# EUROPÄISCHE PATENTANMELDUNG

(11) **EP 0 915 083 A2**
(43) Veröffentlichungstag der Anmeldung: **12.05.1999**
(21) Anmeldenummer: 98119419.4
(22) Anmeldetag: 14.10.1998
(51) Int. Cl.: C07C 277/08, C07C 279/14, C07C 273/18, C07C 275/70

(54) **Verfahren zur Herstellung von substituierten Guanidinderivaten**

(30) Priorität: 04.11.1997 DE 19748696
(71) Anmelder: BASF AKTIENGESELLSCHAFT, 67056 Ludwigshafen (DE)
(72) Erfinder: Greindl, Thomas Dr., 67098 Bad Dürkheim (DE); Scherr, Günter Dr., 67065 Ludwigshafen (DE); Schneider, Rolf Dr., 68163 Mannheim (DE); Mundinger, Klaus Dr., 67117 Limburgerhof (DE)

(57) **Zusammenfassung**

Verfahren zur Herstellung von substituierten Guanidinderivaten der Formel I, 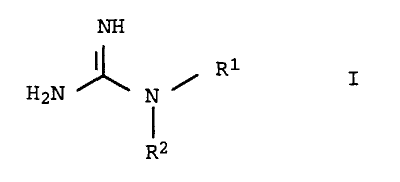 dadurch gekennzeichnet, daß man
a) Harnstoff zu einem alkylierten Isoharnstoff der Formel II, 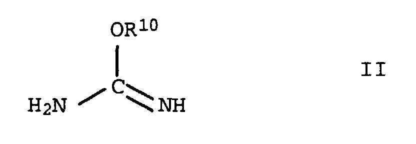 umsetzt, und
b) den alkylierten Isoharnstoff mit einem primären oder sekundären Amin der Formel III, 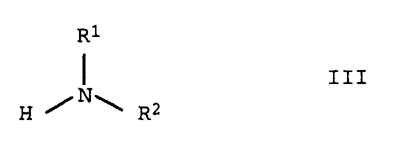 umsetzt, wobei die Substituenten R¹ und R² und R¹⁰ die in der Beschreibung erläuterte Bedeutung haben.

## Beschreibung

Die vorliegende Erfindung betrifft ein Verfahren zur Herstellung von substituierten Guanidiniumverbindungen durch Reaktion von Harnstoff mit Dialkylsulfaten zu Isoharnstoffderivaten und deren Umsetzung mit primären oder sekundären Aminen zu substituierten Guanidiniumverbindungen.

Substituierte Guanidiniumverbindungen sind in der Natur weit verbreitet. Wichtige Vertreter dieser Substanzklasse sind beispielsweise Aminosäuren, wie Arginin und Kreatin. Darüberhinaus sind substituierte Guanidinverbindungen als sterisch gehinderte Basen, als Biozide sowie als Komplexliganden bekannt. Die Mehrzahl der Verbindungen dieses Typs sind aufgrund der hohen Herstellkosten in ihrer technischen Anwendbarkeit jedoch stark eingeschränkt.

Ein Beispiel für ein biologisch aktives Guanidinderivat ist Kreatin, das als "Energieträger der Zelle" zur Nahrungsergänzung im Food und Pharmabereich eingesetzt wird.

Die Herstellung von Kreatin wird z.B. in EP-A-0 754 679 und der dort zitierten weiterführenden Literatur beschrieben, wobei maximale Ausbeuten von lediglich 70% erzielt werden.

Ein Nachteil der o.g. Synthesen für Guanidiniumverbindungen ist die Verwendung wäßriger Lösungen von reinem Cyanamid. Diese Lösungen sind sehr teuer und aufgrund der Instabilität des Cyanamids im allgemeinen nicht breit verfügbar.

Die Synthese von Guanidiniumsalzen, ausgehend von reinen O-Alkylisoharnstoffderivaten, ist beschrieben von R. B. Fearing and S. W. Fox in J. Am Chem. Soc. 76 (1954) 4302-4385.

Die Umsetzung von Sarkosin mit O-Methylisoharnstoff-Hydrochlorid, beschrieben von E. Schütte in Hoppe-Seylers Z. Physiol. Chemie 279 (1943) 52-59, liefert Kreatin in nur 21% Ausbeute.

JP 077364 beschreibt die Umsetzung von einer Lösung von Na-Sarkosinat mit O-Methylisoharnstoff-Methylsulfat bei pH 11 zu Kreatin.

Gemeinsames Merkmal der o.g. Guanidiniumsynthesen ist die Verwendung von reinen Einsatzstoffen.

Die Herstellung von O-Alkylisoharnstoffen ist durch säurekatalysierte Umsetzung von wasserfreiem Cyanamid mit Alkoholen bereits beschrieben (H. Krommer, Chem. Ztg. 98 (1974) 617-618; J. Stieglitz, R. H. McKee, Chem. Ber. 33 (1900) 1517-1519).

Ein Nachteil dieser Reaktion besteht in dem Einsatz von teurem und schlecht verfügbarem, wasserfreiem Cyanamid.

Eine weitere Möglichkeit zur Herstellung von O-Alkylisoharnstoffen besteht in der Umsetzung von Harnstoff mit Dialkylsulfaten. So wird in JP 78-77365 die Synthese von O-Methylisoharnstoff durch Alkylierung von Harnstoff mit Dimethylsulfat beschrieben.

N. Heyboer et al. in Recl. Trav. Chim. Pays-Bas, 81 (1962), 69-72, J. W. Janus in J. Chem. Soc. (1955) 3551-3552 sowie D. J. Brown, E. Hoerger in J. Appl. Chem. 4 (1954), 283-284 beschreiben die Synthese von O-Alkyl-Guanidiniumsalzen durch Umsetzung von Harnstoffs mit Dialkylsulfaten ohne Verwendung von Lösungsmitteln. Die erhaltenen Ausbeuten liegen hier deutlich unter 50%.

In all den o.g. Fällen wird dabei das Gemisch aus Harnstoff und Dialkylsulfat komplett vereinigt und auf eine bestimmte Temperatur gebracht. Aufgrund der schlechten Löslichkeit des Harnstoffes im Reaktionsansatz, kommt es während der Reaktion zu einem ständigen Nachlösen von Harnstoff, was zu einem stetigen Temperaturanstieg des Reaktionsgemisches führt und in der Regel nur durch eine komplizierte Reaktionsführung unterbunden werden kann.

Nachteilig bei den o.g. Verfahren ist außerdem das Auftreten unerwünschter N-Alkylierungen und Mehrfachalkylierungen, was bei der nachfolgenden Umsetzung zur Guanidinverbindung zu schlechten Ausbeuten und ungenügender Reinheit des Wertproduktes führt. Zudem entsteht besonders bei Umsetzungen im technischen Maßstab wegen der, mit Verzögerung anspringenden, stark exothermen Reaktion ein erhebliches Sicherheitsproblem.

Es bestand daher die Aufgabe, ein kostengünstiges und einfach durchführbares Verfahren zur Herstellung substituierter Guanidine auf Basis breit verfügbarer Einsatzstoffe bereitzustellen, das die oben genannten Nachteile nicht aufweist.

Diese Aufgabe wurde gelöst durch ein Verfahren zur Herstellung von substituierten Guanidinderivaten der Formel I, 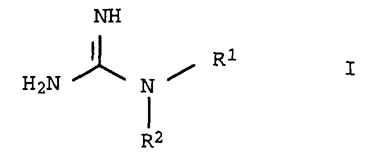 bei der die Substituenten R¹ und R² unabhängig voneinander folgende Bedeutung haben:
- R¹: H,
C₁-C₂₀-Alkyl, C₂-C₁₀-Alkenyl, C₃-C₈-Cycloalkyl;
- R²: C₁-C₂₀-Alkyl, C₂-C₁₀-Alkenyl, C₃-C₈-Cycloalkyl, ―(C₁-C₂₀-Alkylen)―COOR³, ―C₁-C₂₀-Alkylen)―CONR⁴R⁵, ―(C₁-C₂₀-Alkylen)―CN, ―(C₁-C₂₀-Alkylen)―SO₂R⁶, ―[(CH₂)ₘ―X―]ₚ―[(CH₂)ₙ―Y―]_{q}―[(CH₂)₀]ᵣ―Z;
- m, n, o: 0 bis 10;
- p, q, r: 0 bis 50000;
- X: O, NH;
- Y: N―[(CH₂)ₘ―X―]ₚ―[(CH₂)ₙ―Y―]_{q}―[(CH₂)ₒ]ᵣ―Z;
- Z: OH, NH₂;
- R³: H, C₁-C₂₀-Alkyl, C₂-C₁₀-Alkenyl, C₃-C₈-Cycloalkyl, C₆-C₁₈-Aryl,
Na, K, Li, Ca, Mg, N(R⁷)₄;
- R⁴ und R⁵: unabhängig voneinander
H, C₁-C₂₀-Alkyl, C₂-C₁₀Alkenyl, C₃-C₈-Cycloalkyl, C₆-C₁₈-Aryl;
- R⁶: OR⁸, N(R⁹)₂;
- R⁷: H, C₁-C₂₀-Alkyl;
- R⁸: H, C₁-C₂₀-Alkyl, C₂-C₁₀-Alkenyl, C₃-C₈-Cycloalkyl, C₆-C₁₈-Aryl,
Na, K, Li, Ca, Mg, N(R⁷)₄;
- R⁹: H, C₁-C₂₀-Alkyl, C₂-C₁₀-Alkenyl, C₃-C₈-Cycloalkyl, C₆-C₁₈-Aryl,
dadurch gekennzeichnet, daß man
a) Harnstoff zu einem alkylierten Isoharnstoff der Formel II, 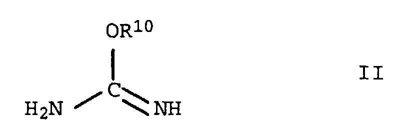 umsetzt, wobei R¹⁰ C₁-C₂₀-Alkyl bedeuten kann,
b) den alkylierten Isoharnstoff II mit einem primären oder sekundären Amin der Formel III, 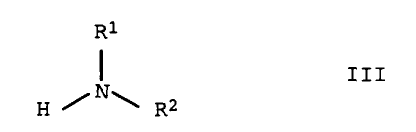 bei der die Substituenten R¹ und R² die oben genannte Bedeutung haben, zu den substituierten Guanidinverbindungen der Formel I umsetzt.

Das erfindungsgemäße Verfahren erfüllt insbesondere wirtschaftliche Randbedingungen, wie niedrige Einsatzstoffkosten, technisch einfache Durchführbarkeit sowie verbesserte Ausbeuten und hinreichende Reinheit des Produktes.

Das Verfahren zeichnet sich besonders durch eine Kombination billiger Einsatzstoffe aus. So wird in der ersten Stufe für die Herstellung von O-Alkylisoharnstoffen, anstelle des beispielsweise teueren reinen Cyanamids, sehr billiger und breit verfügbarer Harnstoff eingesetzt. Im zweiten Verfahrensschritt wird, anstelle von reinem O-Methylisoharnstoff-Sulfat bzw. O-MethylisoharnstoffHydrogensulfat oder O-Methylisoharnstoff-Hydrochlorid, technischer, nicht aufgereinigter O-Alkylisoharnstoff verwendet.

Nach dem erfindungsgemäßen Verfahren können die alkylierten Isoharnstoffderivate der Formel II durch Umsetzung von Harnstoff mit den in der Literatur üblichen Alkylierungsmitteln, wie beispielsweise Alkylhalogeniden der Formel C₁-C₂₀-X (X = Cl, Br, J) oder bevorzugt Dialkylsulfate der Formel (R¹⁰)₂O-SO₂ hergestellt werden.

So lassen sich die alkylierten Isoharnstoffderivate der ersten Synthesestufe durch Zugabe von Dialkylsulfat der Formel (R¹⁰)₂O-SO₂ zu einer Mischung aus a) 0,01 bis 1 Äquivalenten, bevorzugt 0,01 bis 0,8 Äquivalenten, besonders bevorzugt 0,01 bis 0,5 Äquivalenten, bezogen auf Harnstoff, einer Säure, beispielsweise organische Säuren wie p-Toluolsulfonsäure, Methylsulfonsäure, Essigsäure, Chloressigsäure, Trifluoressigsäure sowie bevorzugt Mineralsäuren wie HC1, H₂SO₄, HBF₄ und H₃PO₄ und b) Harnstoff herstellen.

Im Falle der Mineralsäuren können insbesondere auch Gemische, wie z.B. Salzsäure/Schwefelsäure oder Salzsäure/Phosphorsäure in einem Verhältnis von 20/1 bis 5/1, insbesondere 15/1 bis 8/1 verwendet werden.

Als Dialkylsulfate kommen solche Verbindungen in Frage, bei denen R¹⁰ verzweigte oder unverzweigte C₁-C₂₀-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethyl-butyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methyl-propyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl bedeuten kann.

Besonders bevorzugte Dialkylsulfate sind solche, bei denen R¹⁰ lineare oder verzweigte aliphatische Reste mit 1 bis 6 Kohlenstoffatome, wie z.B. Methyl, Ethyl, Propyl, Butyl, Pentyl oder Hexyl bedeutet.

Als Lösungsmittel können u.a. dipolar, aprotische Lösungsmittel oder auch Wasser und Alkohole, bevorzugt Methanol oder Ethanol eingesetzt werden. Die Konzentration an Harnstoff liegt dabei im Bereich von 10 bis 99 Gew.-%, bevorzugt im Bereich von 30 bis 99 Gew.-%, besonders bevorzugt im Bereich von 50 bis 99 Gew.-%.

Es kann aber auch ganz auf das Lösungsmittel verzichtet werden und nur die Säure zugesetzt werden, ggf. können Emulgatoren wie alkylierte Polyether in geringen Mengen zugesetzt werden.

Bei der Alkylierung mit Dialkylsulfat ohne Zugabe eines weiteren Lösungsmittels liegt der entstandene O-Alkyl-Isoharnstoff in flüssiger Phase vor und dient somit gleichzeitig als Lösungsmittel bzw. Lösungsvermittler für weiter zugesetzten Harnstoff, so daß gegebenenfalls nachfolgend durchgeführte Alkylierungsreaktionen in einem einphasigen, flüssigen System erfolgen können.

Die Zugabe von Dialkylsulfat zum Harnstoff erfolgt in gleichmäßigen Portionen über einen Zeitraum von 0,5 bis 10 Std., bevorzugt von 1 bis 6 Std., besonders bevorzugt über einen Zeitraum von 2 bis 5 Std.

Das Molverhältnis von Harnstoff zu Dialkylsulfat liegt im Bereich von 1:0,5 bis 1:1,5, vorzugsweise im Bereich von 1:0,7 bis 1:1,2, besonders bevorzugt im Bereich von 1:0,8 bis 1:1.

Die Reaktionstemperatur liegt dabei im Bereich von -20 bis 90°C, bevorzugt im Bereich von -10 bis 80°C, insbesondere im Bereich von 0 bis 75°C.

Nach der Dosierung wird in der Regel 0,5 bis 10 Std., bevorzugt 1 bis 5 Std. nachgerührt.

Durch die im ersten Schritt des erfindungsgemäßen Verfahrens eingeführten Reaktionsparametern, wie die Zugabe einer Säure, Durchführung der Alkylierung bei niedrigen Temperaturen sowie einer Semi-batch Fahrweise, bei der Dialkylsulfat zum Harnstoff zudosiert wird, ist es gelungen, Ausbeute und Selektivität bei der Alkylierung von Harnstoff zu erhöhen.

Der im Verfahrensschritt a) gebildete O-Alkylisoharnstoff läßt sich ohne weitere Aufreinigung in einer zweiten Verfahrensstufe mit primären oder sekundären Aminen zu substituierten Guanidinverbindungen umsetzen.

Geeignet für die Umsetzung mit den Isoharnstoffderivaten der Formel II sind prinzipiell alle beanspruchten Amine der Formel III. Dabei kann es sich sowohl um aliphatische oder cycloaliphatische primäre bzw. sekundäre Amine handeln, als auch um Aminocarbonsäuren und Aminosulfonsäuren und deren Derivate. Weiterhin lassen sich mit dem erfindungsgemäßen Verfahren auch primäre und sekundäre Amine umsetzen, die zusätzliche Amino- oder Iminogruppen enthalten, sowie aminogruppenhaltige Oligomere bzw. Polymere.

Als Alkylreste für R¹ bis R⁵ sowie für R⁷ bis R⁹ seien verzweigte oder unverzweigte C₁-C₂₀-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als Alkenylreste für R¹ bis R⁵ sowie für R⁸ und R⁹ seien verzweigte oder unverzweigte C₂-C₁₀-Alkenylketten, bevorzugt Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 2-Hexenyl, 1-Heptenyl, 2-Heptenyl, 1-Octenyl oder 2-Octenyl genannt.

Als Alkylenreste für R² seien verzweigte oder unverzweigte C₁-C₂₀-Alkylenketten, bevorzugt Methylen, Ethylen, n-Propylen, 1-Methylethylen, n-Butylen, 1-Methylpropylen-, 2-Methylpropylen, 1,1-Dimethylethylen, n-Pentylen, 1-Methylbutylen, 2-Methylbutylen, 3-Methylbutylen, 2,2-Dimethylpropylen, 1-Ethylpropylen, n-Hexylen, 1,1-Dimethylpropylen, 1,2-Dimethylpropylen, 1-Methylpentylen, 2-Methylpentylen, 3-Methylpentylen, 4-Methylpentylen, 1,1-Dimethylbutylen, 1,2-Dimethylbutylen, 1,3-Dimethylbutylen, 2,2-Dimethylbutylen, 2,3-Dimethylbutylen, 3,3-Dimethylbutylen, 1-Ethylbutylen, 2-Ethylbutylen, 1,1,2-Trimethylpropylen, 1,2,2-Trimethylpropylen, 1-Ethyl-1-methylpropylen, 1-Ethyl-2-methylpropylen, n-Heptylen, n-Octylen, n-Nonylen, n-Decylen, n-Undecylen, n-Dodecylen, n-Tridecylen, n-Tetradecylen, n-Pentadecylen, n-Hexadecylen, n-Heptadecylen, n-Octadecylen, n-Nonadecylen oder n-Eicosylen genannt.

Die 1- bis 20-gliedrigen Alkylenketten können optional mit folgenden Resten substituiert sein:
C₁-C₆-Alkyl, beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl;
Sulfhydrylmethyl, 1-Aminobutyl, 1-Carboxyethyl;
Arylalkyl, beispielsweise Benzyl, p-Hydroxybenzyl, Indolylmethyl.

Als Cycloalkylreste seien für R¹ bis R⁵ sowie für R⁸ und R⁹ verzweigte oder unverzweigte C₃-C₈-Cycloalkylketten, bevorzugt Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclypropyl, 1-Methyl-2-Ethylcyclopropyl oder Cyclooctyl genannt.

Die Cycloalkylreste können ggf. mit einem oder mehreren, z.B. 1 bis 3 Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein oder 1 bis 3 Heteroatome wie Schwefel, Stickstoff, dessen freie Valenzen durch Wasserstoff oder C₁-C₄-Alkyl abgesättigt sein können oder Sauerstoff im Ring enthalten.

Als Alkoxyreste für R⁶ kommen bevorzugt solche mit 1 bis 20 C-Atomen, vorzugsweise mit 1 bis 12 C-Atomen, besonders bevorzugt 1 bis 8 C-Atomen in Betracht.

Beispielsweise sind zu nennen:
- Methoxy-: Ethoxy-
- Isopropoxy-: n-Propoxy-
- 1-Methylpropoxy-: n-Butoxy-
- n-Pentoxy-: 2-Methylpropoxy-
- 3-Methylbutoxy-: 1,1-Dimethylpropoxy-
- 2,2-Dimethylpropoxy-: Hexoxy-
- 1-Methyl-1-ethylpropoxy-: Heptoxy-
- Octoxy-: 2-Ethylhexoxy-

Als mono- oder disubstituierte Aminoreste für R⁶ kommen bevorzugt solche in Betracht, die Alkylreste mit 1 bis 20, bevorzugt 1 bis 12 C-Atomen enthalten, wie z.B. Methyl-, n-Propyl-, n-Butyl-, 2-Methylpropyl-, 1,1-Dimethylpropyl-, Hexyl-, Heptyl-, 2-Ethylhexyl-, Isopropyl-, 1-Methylpropyl-, n-Pentyl-, 3-Methylbutyl-, 2,2-Dimethylpropyl-, 1-Methyl-1-ethylpropyl- und Octyl.

Als Tetraalkylammoniumreste für R³ und R⁸ kommen solche in Betracht, die Alkylreste mit 1 bis 20, bevorzugt 1 bis 12, besonders bevorzugt 1 bis 6 C-Atomen enthalten, wie z.B. Methyl-, n-Propyl-, Isopropyl-, 2-Methylpropyl-, 1,1-Dimethylpropyl-, 1-Methylpropyl-, 2,2-Dimethylpropyl-, 1-Methyl-1-ethylpropyl-, n-Butyl-, 3-Methylbutyl-, n-Pentyl- und Hexyl-.

Unter Aryl sind aromatische Ringe oder Ringsysteme mit 6 bis 18 Kohlenstoffatomen im Ringsystem zu verstehen, beispielsweise Phenyl oder Naphthyl, die ggf. mit einem oder mehreren Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein können. Bevorzugt sind ggf. substituiertes Phenyl, Methoxyphenyl und Naphthyl.

Als bevorzugt verwendete Amine sind alle die in Wasser oder in, mit Wasser mischbaren Lösungsmitteln löslichen, primären und sekundären Amine genannt. Bevorzugte Vertreter unter den einfachen Aminen sind u.a. Methylamin, Ethylamin, n-Propylamin, 2-Propylamin, Butylamin, Isobutylamin, Anilin, Benzylamin und Anthranilsäure. Weitere bevorzugt eingesetzte Aminogruppenhaltige Verbindungen sind u.a. Taurin und Aminocarbonsäuren wie Glycin, Alanin, Valin, Prolin, Leucin, Phenylalanin, Lysin, Methionin Cystein, Asparaginsäure, Iminodiessigsäure, Sarkosin sowie deren Ester, Amide und Nitrile und deren Salze.

Ganz besonders bevorzugte Verbindung der Formel III ist Sarkosin, das sowohl als freie Säure als auch insbesondere als Na- oder K-Salz in Form einer 5 bis 60 Gew.-%igen, bevorzugt 35 bis 45 Gew. -%igen wäßrigen Lösung verwendet werden kann.

In dem erfindungsgemßen Verfahren können auch wasserlösliche, Aminogruppen enthaltende Oligomere und Polymere eingesetzt werden, wie Alkylendiamine, Dialkylentriamine bis hin zu Polyalkylenpolyamine oder Polyetherdiamine. Bevorzugte Vertreter dieser Gruppe sind Ethylendiamin, Propylendiamin, Butylendiamin, Hexamethylendiamin, Diethylentriamin, Triethylentetramin, Tetraethylenpentamin, Pentaethylenhexamin, Hexaethylenheptamin sowie verzweite oder lineare Polyalkylenpolyamine.

Von den Polyalkylenpolyaminen kommen vorzugsweise Polyethylenimine in Betracht, die beispielsweise Molmassen von 200 bis 10 Millionen, vorzugsweise 1000 bis 3 Millionen haben. Besonders bevorzugt werden Polyethylenimine mit Molmassen von 2000 bis 1300000 eingesetzt.

Die Polyetherdiamine werden beispielsweise durch Umsetzung von Polyalkylenglykolen mit Ammoniak hergestellt. Die Polyalkylenglykole können 2 bis 50, vorzugsweise 2 bis 40 Alkylenoxideinheiten enthalten. Hierbei kann es sich beispielsweise um Polyethylenglykole, Polypropylenglykole, Polybutylenglykole oder auch um Blockcopolymerisate aus Ethylenglykol und Propylenglykol, Blockcopolymerisate aus Ethylenglykol und Butylenglykol oder um Blockcopolymerisate aus Ethylenglykol, Propylenglykol und Butylenglykol handeln. Außer den Blockcopolymerisaten eignen sich zur Herstellung der Polyetherdiamine statistisch aufgebaute Copolymerisate aus Ethylenoxid und Propylenoxid und gegebenenfalls Butylenoxid. Polyetherdiamine leiten sich außerdem von Polytetrahydrofuranen ab, die 2 bis 75 Tetrahydrofuraneinheiten aufweisen. Die Polytetrahydrofurane werden ebenfalls durch Umsetzung mit Ammoniak in die entsprechenden α,ω-Polyetherdiamine überführt. Vorzugsweise verwendet man zur Herstellung der Polyetherdiamine Polyethylenglykole oder Blockcopolymerisate aus Ethylenglykol und Propylenglykol.

Weitere geeignete Aminogruppen enthaltende wasserlösliche Polymere sind Polyvinylamine, die erhältlich sind durch Homo- und/oder Copolymerisation von N-Vinylformamid und anschließende Hydrolyse der Polymerisate sowie Vinylamineinheiten enthaltenden Polymeren. Stoffe dieser Art sind bekannt, vgl. EP-B-0 071 050 und EP-B-0 216 387. Bevorzugt in Betracht kommende Polymerisate sind hydrolysierte Homopolymerisate des N-Vinylformamid mit einem Hydrolysegrad von 1 bis 100, vorzugsweise 80 bis 100 % und partiell oder vollständig hydrolysierte Copolymerisate aus N-Vinylformamid und Vinylformiat oder Vinylacetat. Das in die Copolymerisate einpolymerisierte N-Vinylformamid ist vorzugsweise zu 80 bis 100 % hydrolysiert. Je nach Hydrolysebedingungen können die einpolymerisierten Monomeren wie Vinylformiat oder Vinylacetat ganz oder vollständig zu Vinylalkohol-Einheiten hydrolysiert sein. Weitere Comonomere, die für die Herstellung von hydrolysierten Copolymerisaten des N-Vinylformamids in Betracht kommen, sind monoethylenisch ungesättigte Carbonsäuren wie Acrylsäure, Methacrylsäure oder Maleinsäure, N-Vinylpyrrolidon und Acrylnitril.

Weitere Aminogruppen enthaltende wasserlösliche Polymere sind Polyallylamine. Diese Polymeren enthalten mindestens 3 Allylamin-Einheiten einpolymerisiert und haben Molmassen bis zu 10 Millionen.

Der Einsatz von technischen Produkten in dem erfindungsgemäßen Verfahren empfiehlt sich insbesondere dort, wo keine weiteren unerwünschten reaktiven Amine beigemischt sind und es aus wirtschaftlichen Gründen besonders vorteilhaft ist, weil beispielsweise die Aufreinigung des Amins teuer und aufwendig ist.

Die Umsetzung der substituierten Isoharnstoffderive mit den o.g. Aminen kann in Wasser oder einem mit Wasser mischbaren Lösungsmittel, oder einem Gemisch davon erfolgen. Üblicherweise wird dabei ein pH-Wert im Bereich des pK-Wertes des Amins verwendet, d.h. in einem pH-Bereich zwischen 6 und 14, bevorzugt zwischen 8 und 12, besonders bevorzugt im pH-Bereich von 9 bis 11,5.

Das Molverhältnis von O-Alkylisoharnstoff zum primären oder sekundären Amin liegt im Bereich von 0,9 bis 5,0, vorzugsweise im Bereich von 1,0 bis 2,0.

Die Reaktionstemperaturen in der zweiten Stufe liegen im Bereich von -20 bis 100°C, bevorzugt im Bereich von 0 bis 80°C, besonders bevorzugt zwischen 10 und 60°C.

Für die Reaktionsführung in der zweiten Verfahrensstufe spielt die Reihenfolge der Zugabe der Reaktanten keine besondere Bedeutung. In der Regel gibt man den substituierten Isoharnstoff zu dem primären oder sekundären Amin, der bevorzugt in wäßriger oder alkoholischer Lösung vorliegen kann.

Die Zugabe kann sich über einen Zeitraum von 0,5 bis 10 Std., bevorzugt von 1 bis 3 Std. erstrecken.

Zur Einhaltung des pH-Wertes können je nach Ausgangs-pH-Wert der Base entweder Säuren, wie CO₂, SO₂, HCl, HNO₃, H₂SO₄, H₂SO₃, H₃PO₃, H₃PO₂ und H₃PO₄, und/oder Basen wie NaOH, KOH, LiOH, Ca(OH)₂, Ba(OH)₂, Mg(OH)₂ eingesetzt werden. Sollten die Amine in basischer und nicht in neutralisierter oder teilneutralisierter Form vorliegen, so werden nur Säuren benötigt.

Als Säuren bevorzugt sind z.B. CO₂; H₂SO₄, H₃PO₄. Es können aber auch bevorzugt Gemische dieser und anderer Säuren eingesetzt werden.

Die Isolierung der gewünschten Guanidiniumderivate in an sich bekannter Weise. So läßt sich beispielsweise für Kreatin durch Abkühlen der abfiltrierten Reaktionslösung auf -20 bis 60°C, insbesondere 0 bis 40°C, das Wertprodukt kristallin erhalten. Nach Filtration kann gegebenenfalls durch eine weitere Umkristallisation die Reinheit verbessert werden. Es ist aber auch möglich, daß Produkt mittels Extraktion aus dem Reaktionsgemisch zu entfernen, um es anschließend durch Destillation oder Kristallisation sauber zu isolieren.

Es ist in besonderer Weise überraschend, daß die Ausbeuten der erfindungsgemäßen Reaktion, bezogen auf den Gehalt an Cyanamid (1. Stufe) und O-Alkylisoharnstoff (2. Stufe) bei der Verwendung der technischen Ausgangsstoffe mit denen der Umsetzung von reinem Cyanamid und O-Alkylisoharnstoffen vergleichbar sind. Unter Berücksichtigung des Reinigungsschrittes für die jeweilige Herstellung von reinem Cyanamid bzw. O-Alkylisoharnstoff liegt die Ausbeute aufgrund der geringeren Zahl der Verfahrensschritte weit höher.

Zudem können noch höhere Umsätze erzielt werden, wenn man im zweiten Verfahrensschritt die billige Isoharnstoffverbindung im Überschuß zum Amin einsetzt, was bei Einsatz von reinem O-Alkylisoharnstoff häufig unwirtschaftlich ist. Die erreichte Reinheit des isolierten Guanidiniumsalzes ist mit der aus reinem O-Alkylisoharnstoff hergestellten Ware vergleichbar. Dies ist insbesondere auf die hohe Reinheit der erfindungsgemäß erhaltenen Isoharnstoffderivate zurückzuführen.

In dem folgenden Beispiel wird das Verfahren zur Herstellung substituierter Guanidiniumderivate näher erläutert.

### Beispiel 1: Herstellung von Kreatin

### Stufe 1:

### Synthese von technischem O-Methylisoharnstoff

In einem 250 ml Dreihalskolben mit mechanischem Rührer, Heizbad, Rückflußkühler und Trockenrohr wurden 66 g Harnstoff mit 2 ml trockenem Methanol und 2 ml Methansulfonsäure versetzt und anschließend auf 40°C erhitzt. Sofort nach Erreichen der Temperatur wurde über eine Dosierpumpe, mit einer Rate von 126 g pro h, 1 mol Dimethylsulfat zudosiert. Während des Zudosierens begann sich die Reaktionsmischung zu erwärmen und wurde unter Eiskühlung auf eine Temperatur von 70°C gehalten. Nach Ende des Zudosierens wurde noch 2h bei 70°C weitergerührt. Nach Abkühlen verblieben 196 g eines farblosen, dünnflüssigen Öls, das laut HPLC 92% O-Methylisoharnstoff Methylsulfat enthielt, entsprechend einer Ausbeute von 97%.

Der Gehalt an N-Methyl-O-Methyl-isoharnstoff Methylsulfat betrugt 0,51 Gew.-%, entsprechend einer Ausbeute von 0,5 %, der Gehalt an Harnstoff betrug 5,2 Gew.-%.

### Stufe 2:

### Umsetzung von Sarkosin-Na-Salz-Lösung mit technischem O-Methylisoharnstoff bei pH 11

In einem 2l-Rührreaktor mit Rückflußkühler, Blattrührer, Thermostat und pH-gesteuerter Schwefelsäuredosierung und Natronlaugedosierung wurden zu einem Gemisch von 138 g 40, 1%-iger wässriger Na-Sarkosinat-Lösung und 53 g Wasser zur Einstellung eines pH-Wertes von 11,0 insgesamt 16,2 g 50%-ige Schwefelsäure zugegeben. Anschließend wurden mit Hilfe einer waagengesteuerten Dosierpumpe bei einer Temperatur von 20°C über einen Zeitraum von 2h kontinuierlich 146 g des 92%igen methanolischen O-Methyl-isoharnstoffmethylsulfats aus Stufe 1 zudosiert. Dabei wurde der pH-Wert durch Zugabe von 143 g 25%iger wässriger Natronlauge bei 11,0 gehalten. Bereits während des Zudosierens begann Kreatin auszufallen. Nach Ende der Zugabe wurde noch 6 h bei 20⁰C nachgerührt. Nach dieser Zeit war kein alkylierendes Potential (Preußmann-Test) mehr nachweisbar. Anschließend wurde noch für 2h auf 0 bis 5⁰C abgekühlt. Nach Filtration der gebildeten farblosen Kristalle und Waschen mit 2 x 50 ml Wasser, und anschließender Trocknung im Vakuum bei 60°C werden 63,3 g Kristalle mit einem Kreatingehalt von 88% und einem Restwassergehalt von 12% erhalten, entsprechend einer isolierten Ausbeute von 85%.

Der Gehalt an Kreatin in der Mutterlauge lag bei 1,1%, damit errechnet sich eine Ausbeute der Reaktion von 94%.

### Beispiel 2: Herstellung von Kreatin

### Stufe 1:

### Synthese von technischem O-Methyl-isoharnstoff

In einem 250 ml Dreihalskolben mit mechanischem Rührer, Heizbad, Rückflußkühler und Trockenrohr wurden 66 g Harnstoff mit 2 ml 50%-iger wässriger Schwefelsäure versetzt und anschließend auf 40°C erhitzt. Sofort nach Erreichen der Temperatur wurde über eine Dosierpumpe, mit einer Rate von 126 g pro h, 1 mol Dimethylsulfat zudosiert. Während des Zudosierens begann sich die Reaktionsmischung zu erwärmen und es mußte mit Eis gekühlt werden, um ein Ansteigen der Temperatur über 70°C zu vermeiden. Nach Ende des Zudosierens wurde noch 2h bei 70°C weitergerührt. Nach Abkühlen verblieben 194 g eines farblosen, dünnflüssigen Öls, das laut HPLC 90 Gew.-% O-Methylisoharnstoff Methylsulfat enthielt, entsprechend einer Ausbeute von 94%.

Der Gehalt an N-Methyl-O-Methyl-isoharnstoff Methylsulfat betrug 0,47%, entsprechend einer Ausbeute von 0,5%, der Gehalt an Harnstoff betrug 5,7%.

### Stufe 2:

### Umsetzung von Sarkosin-Na-Salz-Lösung mit technischem O-Methylisoharnstoff

In einem 2l-Rührreaktor mit Rückflußkühler, Blattrührer, Thermostat und pH-gesteuerter Schwefelsäuredosierung und Natronlaugedosierung wurden zu einem Gemisch von 138 g 40,1%-iger wässriger Na-Sarkosinat-Lösung zur Einstellung eines pH-Wertes von 11,0 insgesamt 17,2 g 37%-ige Salzsäure zugegeben. Anschließend wurden mit Hilfe einer waagengesteuerten Dosierpumpe bei einer Temperatur von 20°C über einen Zeitraum von 2h kontinuierlich 149 g des 90%igen O-Methyl-isoharnstoff-methylsulfats aus Stufe 1 zudosiert. Dabei wurde der pH-Wert durch Zugabe von 137 g 25%iger wässriger Natronlauge bei 11,0 gehalten. Bereits während des Zudosierens begann Kreatin auszufallen. Nach Ende der Zugabe wurde noch 6 h bei 20°C nachgerührt. Nach dieser Zeit war kein alkylierendes Potential (Preußmann-Test) mehr nachweisbar. Anschließend wurde noch für 2h auf 0 - 5°C abgekühlt. Nach Filtration der gebildeten farblosen Kristalle und Waschen mit 2 x 50 ml Eiswasser, und anschließender Trocknung im Vakuum bei 60°C wurden 65,7 g Kristalle mit einem Kreatingehalt von 87% und einem Restwassergehalt von 12% erhalten, entsprechend einer isolierten Ausbeute von 87%.

Der Gehalt an Kreatin in der Mutterlauge lag bei 0,8%, damit errechnet sich eine Ausbeute der Reaktion von 93%.

### Beispiel 3: Herstellung von Kreatin

### Stufe 1:

### Synthese von technischem O-Methyl-isoharnstoff

In einem 250 ml Dreihalskolben mit mechanischem Rührer, Heizbad, Rückflußkühler und Trockenrohr wurde ein Gemisch aus 126 g Dimethylsulfat und 1 g 50%-iger wässriger Schwefelsäure bei 70°C portionsweise mit 60 g Harnstoff versetzt, sodaß die Temperatur konstant gehalten werden konnte. Nach 30 min war der gesamte Harnstoff gelöst, es wurde noch 1h bei 70°gerührt. Anschließend wurde auf 60°C abgekühlt und im Reaktionsansatz wurden weitere 60 g Harnstoff gelöst. Zu der Lösung wurde über eine Dosierpumpe, mit einer Rate von 126 g pro h, 1 mol Dimethylsulfat zudosiert, während der gesamten Zugabezeit war die Mischung homogen, die Temperatur wurde bei 60°C gehalten. Nach Ende des Zudosierens wurden erneut 60 g Harnstoff zugesetzt und der Dosiervorgang für Dimethylsulfat nach dem Auflösen des Harnstoffs wiederholt. Nach erfolgter Zuage wurde der Vorgang wiederholt, d.h. es wurden nochmals Harnstoff und Dimethylsulfat in der beschriebenen Weise zugegeben.

Nach Abkühlen der klaren Reaktionsmischung verblieben 726 g eines farblosen, dünnflüssigen Öls, das laut HPLC 97% O-Methylisoharnstoff Methylsulfat enthält, entsprechend einer Ausbeute von 95%.

Der Gehalt an N-Methyl-O-Methyl-isoharnstoff Methylsulfat betrug 0,32%, entsprechend einer Ausbeute von 0,3%, der Gehalt an Harnstoff betrug 0,5%.

### Stufe 2:

### Umsetzung von Sarkosin-Na-Salz-Lösung mit technischem O-Methylisoharnstoff

In einem 2l-Rührreaktor mit Rückflußkühler, Blattrührer, Thermostat und pH-gesteuerter Schwefelsäuredosierung und Natronlaugedosierung wurden zu einem Gemisch von 138 g 40,1%-iger wässriger Na-Sarkosinat-Lösung zur Einstellung eines pH-Wertes von 11,0 insgesamt 17,2 g 37%-ige Salzsäure zugegeben. Anschließend wurden mit Hilfe einer waagengesteuerten Dosierpumpe bei einer Temperatur von 40°C über einen Zeitraum von 2h kontinuierlich 120 g des 97%igen O-Methyl-isoharnstoff-methylsulfats aus Stufe 1 zudosiert. Dabei wurde der pH-Wert durch Zugabe von 134 g 25%iger wässriger Natronlauge bei 11,0 gehalten. Bereits während des Zudosierens begann Kreatin auszufallen. Nach Ende der Zugabe wurde noch 2 h bei 40°C nachgerührt. Nach dieser Zeit war kein alkylierendes Potential (Preußmann-Test) mehr nachweisbar. Anschließend wurde noch für 2h auf 0 bis 5°C abgekühlt. Nach Filtration der gebildeten farblosen Kristalle und Waschen mit 2 x 50 ml Eiswasser, und anschließender Trocknung im Vakuum bei 60°C wurden 69,3 g Kristalle mit einem Kreatingehalt von 89% und einem Restwassergehalt von 11% erhalten, entsprechend einer isolierten Ausbeute von 94%.

Der Gehalt an Kreatin in der Mutterlauge lag bei 0,4%, damit errechnet sich eine Ausbeute der Reaktion von 97%.

## Patentansprüche

1. Verfahren zur Herstellung von substituierten Guanidinderivaten der Formel I, 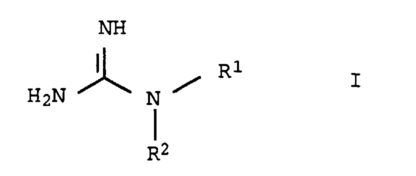 bei der die Substituenten R¹ und R² unabhängig voneinander folgende Bedeutung haben:
R¹ H,
C₁-C₂₀-Alkyl, C₂-C₁₀-Alkenyl, C₃-C₈-Cycloalkyl;
R² C₁-C₂₀-Alkyl, C₂-C₁₀-Alkenyl, C₃-C₈-Cycloalkyl, ―(C₁-C₂₀-Alkylen)―COOR³, ―(C₁-C₂₀-Alkylen)―CONR⁴R⁵, ―(C₁-C₂₀-Alkylen)―CN, ―(C₁-C₂₀-Alkylen)―SO₂R⁶, ―[(CH₂)ₘ―X―]ₚ―[(CH₂)ₙ―Y―]_{q}―[(CH₂)₀]ᵣ―Z;
m, n, o 0 bis 10;
p, q, r 0 bis 50000;
X O, NH;
Y N―[(CH₂)ₘ―X―]ₚ―[(CH₂)ₙ―Y―]_{q}―[(CH₂)₀]ᵣ―Z;
Z OH, NH₂;
R³ H, C₁-C₂₀-Alkyl, C₂-C₁₀-Alkenyl, C₃-C₈-Cycloalkyl, C₆-C₁₈-Aryl,
Na, K, Li, Ca, Mg, N(R⁷)₄;
R⁴ und R⁵ unabhängig voneinander
H, C₁-C₂₀-Alkyl, C₂-C₁₀-Alkenyl, C₃-C₈-Cycloalkyl, C₆-C₁₈-Aryl;
R⁶ OR⁸, N(R⁹)₂;
R⁷ H, C₁-C₂₀-Alkyl;
R⁸ H, C₁-C₂₀-Alkyl, C₂-C₁₀-Alkenyl, C₃-C₈-Cycloalkyl, C₆-C₁₈-Aryl,
Na, K, Li, Ca, Mg, N(R⁷)₄;
R⁹ H, C₁-C₂₀-Alkyl, C₂-C₁₀-Alkenyl, C₃-C₈-Cycloalkyl, C₆-C₁₈-Aryl,
dadurch gekennzeichnet, daß man
a) Harnstoff zu einem alkylierten Isoharnstoff der Formel II, 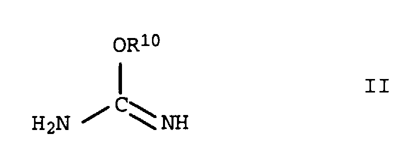 umsetzt, wobei R¹⁰ C₁-C₂₀-Alkyl bedeuten kann,
b) den alkylierten Isoharnstoff II mit einem primären oder sekundären Amin der Formel III, 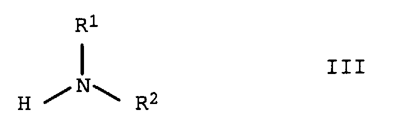 bei der die Substituenten R¹ und R² die oben genannte Bedeutung haben, zu Guanidinverbindungen der Formel I umsetzt.

2. Verfahren nach Anspruch 1, dadurch gekennzeichnet, daß man im Verfahrensschritt a) Harnstoff mit Dialkylsulfat der Formel (R¹⁰O)₂-SO₂ umsetzt.

3. Verfahren nach Anspruch 2, dadurch gekennzeichnet, daß man im Verfahrensschritt a) eine angesäuerte Harnstofflösung oder eine angesäuerte Harnstoffsuspension bei einer Temperatur von -20 bis 90°C mit Dialkylsulfat der Formel (R¹⁰O)₂-SO₂ umsetzt.

4. Verfahren nach den Ansprüchen 2 und 3, dadurch gekennzeichnet, daß man im Verfahrensschritt a) die Umsetzung von Harnstoff mit Dialkylsulfat der Formel (R¹⁰O)₂-SO₂ bei einer Temperatur von -20 bis 90°C ohne Lösungsmittel durchführt.

5. Verfahren nach den Ansprüchen 2 bis 4, dadurch gekennzeichnet, daß man im Verfahrensschritt a) das Dialkylsulfat zum Harnstoff zudosiert.

6. Verfahren nach den Ansprüchen 1 bis 5, dadurch gekennzeichnet, daß man die im Verfahrensschritt a) hergestellten, alkylierten Isoharnstoffderivate ohne Isolierung direkt im Verfahrensschritt b) einsetzt.

7. Verfahren nach den Ansprüchen 1 bis 6, dadurch gekennzeichnet, daß man im Verfahrensschritt b) als primäres oder sekundäres Amin der Formel III ein wasserlösliches oder ein in einem wassermischbaren Lösungsmittel lösliches Amin verwendet.

8. Verfahren nach Anspruch 7, dadurch gekennzeichnet, daß man ein primäres oder sekundäres Amin, ausgewählt aus der Gruppe Aminocarbonsäuren, deren Ester, Amide und Nitrile sowie Aminosulfonsäuren und deren Ester und Amide verwendet.

9. Verfahren nach Anspruch 7, dadurch gekennzeichnet, daß man ein primäres oder sekundäres Amin, ausgewählt aus der Gruppe Alkylendiamine, Dialkylentriamine, Trialkylentetramine und Polyalkylenpolyamine verwendet.
